# EUROPEAN PATENT SPECIFICATION

(11) **EP 1 207 861 B1**
(45) Date of publication and mention of the grant of the patent: **03.08.2005**
(21) Application number: 00961396.9
(22) Date of filing: 29.08.2000
(51) Int. Cl.: A61K 9/70

(54) **TREATMENT OF FUNGAL INFECTIONS UTILIZING A FUNGAL GROWTH MEDIUM**
PILZWACHSTUMSMEDIUM ZUR BEHANDLUNG VON PILZINFEKTIONEN
TRAITEMENT D'INFECTIONS FONGIQUES AU MOYEN D'UN MILIEU DE PROLIFERATION FONGIQUE

(30) Priority: 31.08.1999 US 151570 P
(43) Date of publication of application: 29.05.2002
(73) Proprietor: The Regents of the University of Michigan, Ann Arbor, Michigan (US)
(72) Inventor: PIERARD, Gerald, B-4031 Angleur (BE); PIERARD-FRANCHIMONT, Claudine, B-4031 Angleur (BE); CAUWENBERGH, Geert, Plainsboro, NJ 08536 (US); SUN, Ying, Somerville, NJ 08876 (US)
(74) Representative: Fisher, Adrian John
(86) International application number: PCT/US2000/023659
(87) International publication number: WO 2001/015670

(56) References cited:
- US-A- 5 023 082
- DATABASE WPI Week 198306 Derwent Publications Ltd., London, GB; AN 1983-13037k XP002156352 & JP 57 209211 A (SHISEIDO), 22 December 1982 (1982-12-22)
- PIERARD, GERALD E.; ET AL.: "The boosted oral antifungal treatment for onychomycosis beyond the regular itraconazole pulse dosing regimen" DERMATOLOGY, vol. 200, no. 2, 2000, pages 185-187, XP000978145 basel

## Description

### FIELD OF THE INVENTION

The present invention relates to methods, compositions, and devices (e.g., bandages) for the treatment of topical fungal infections.

### BACKGROUND OF THE INVENTION

The treatment of topical fungal infections has traditionally resulted in variable success. See, e.g., Lauhearanta, Clin. Expert. Dermatol. 17 (supp. 1):41-43 (1992); Reinel, et al., Clin. Exp. Dermatol. 17(supp. 1):44-49 (1992); and Zang, et al., Clin Exp. Dermatol. 17(supp. 1):61-70 (1992). This variability is likely due to the fact that fungi in the growing state (e.g., hyphae) are more susceptible to the toxic action of the antifungal drugs than fungi in resting state (e.g., spores and arthrocomidia). This inability to eradicate all the fungi during antifungal treatment often results in poor treatment efficacy, long treatment duration, and a high recurrence rate.

The present invention relates to a method of improving current antifungal treatment by changing the biological status of fungi from a resting (e.g., dormant) state to the growing (e.g., vegetative) state, thereby increasing the efficacy of antifungal treatment. The conversion of fungi from resting state to growing state is accomplished by topically applying nutrients essential to the growth and reproduction of fungi, either prior to or during the antifungal treatment. The nutrients in the fungal growth medium induces the fungal spores to grow, thereby making them more susceptible to the toxicity of the antifungal agent.

JP57-209211 A discloses a topical composition comprising an antifungal agent and one or more amino acids.

### SUMMARY OF THE INVENTION

In one aspect, the invention features the use of an antifungal agent in the manufacture of a medicament for treating or preventing a topical fungus infection on a mammal (e.g., a human), wherein an amount of a fungal growth medium effective to induce the fungus from the dormant state into the vegetative state is applied to the fungus-infected tissue, and the fungal growth medium comprises a carbon hydrate. The antifungal agent may administered prior to or during the administration of the fungal growth medium. Examples of topical fungal infections include, onychomycosis, tinea capitis, tinea pedis, tinea manuum, and dandruff. In one embodiment, the invention relates to the prevention of fungal infections (e.g., administering the fungal growth medium and antifungal agent to the site of a wound or other topical area susceptible to fungal infection).

In another aspect, the invention features a composition comprising a therapeutically effective amount of an antifungal agent and an amount of a fungal growth medium, comprising a carbon hydrate, capable of inducing fungus from the dormant state into the vegetative state.

In another aspect, the invention features a bandage comprising an amount of a fungal growth medium, comprising a carbon hydrate, capable of inducing fungus from the dormant state into the vegetative state. The bandage may further comprise a therapeutically effective amount of an antifungal agent.

Other features and advantages of the present invention will be apparent from the detailed description of the invention and from the claims

### DETAILED DESCRIPTION OF THE INVENTION

The following specific embodiments are to be construed as merely illustrative, and not limitative of the claims.

Unless defined otherwise, all technical and scientific terms used herein have the same meaning as commonly understood by one of ordinary skill in the art to which the invention belongs.

The present invention relates to the finding that the presence of large loads of spores and arthrocomidia at the infection site was a good predictor of future therapeutic failure and/or frequent recurrence of the fungal infection. In fact, we found that resting fungal cells were almost completely non-susceptible to the toxic action of current oral and topical antifungal agents. We, however, also found that the application of a fungal growth medium would help transform dormant fungal cells into growing hyphae, and, in this growing state, the fungi were more readily susceptible to antifungal treatment.

What is meant by "fungal growth medium" is a composition that is capable of stimulating the transformation of dormant fungi (e.g., spores and arthrocomidia) in to growing fungi (e.g., hyphae). In one embodiment, the growth medium comprises 0.1 to 20% (e.g., between 1 and 10%), by weight, of a carbon hydrate. Examples of carbon hydrates include monosaccharides such as glucose, fructose, mannose, ribose and galactose, disaccharides such as sucrose, maltose and lactose, polysaccharides such as dextrins, dextrans, and starches such as amylose and amylopectin.

In one embodiment, the growth medium further comprises 0.1 to 20% (e.g., between 0.1 and 10%), by weight, of a nitrogen-containing compound. Examples of nitrogen-containing compounds include amino acids and compounds comprising amino acids such as peptides, polypeptides, proteins, and fragments thereof (e.g., a peptone broth and other partially or fully hydrolyzed proteins of animal and plant origins). Other examples of nitrogen-containing compounds include nitrates, ammonia, and ureas.

In one embodiment, the growth medium comprises between 0.05 to 20 % (e.g., between 0.1 and 5%), by weight, of a salt. Examples of salts include sodium chloride, potassium chloride, and lithium chloride.

In one embodiment, the growth medium further comprises a pH adjusting agent (e.g., an acidic, basic, or buffering agent). Examples of acidic agents include hydrochloric acid, citric acid, lactic acid, glycolic acid, salicylic acid, and phosphoric acid. Examples of basic agents include sodium hydroxide, potassium hydroxide and ammonium hydroxide. Examples of buffering agents include phosphate buffers and citrate buffers. Other such pH adjusters and buffering agents are listed in the International Cosmetic Ingredient Dictionary and Handbook, eds. Wenninger and McEwen p. 1625 and 1653 (7^{th} ed. 1997). In one embodiment, the growth medium has a pH of between pH 3 and about pH 7 (e.g., a pH 4 and pH 6 such as a pH of 5.6).

In one embodiment, the growth medium comprises a supporting matrix to provide a solid (e.g., gauzes and non-woven pads) or semi-solid (e.g., gelling agents) structure to the growth medium. Examples of gelling agents include agar, gelatin, pectins, alginates, gums (e.g., karaya gum, gum arabic, tragacanth gum, carrageenan gum, guar gum, gum ghatti, locust bean gum, tamarind gum and xanthan gum), hydrophilic cellulose polymers (e.g., hydroxymethylcellulose, hydroxyethylcellulose, hydroxypropylcellulose, and carboxymethylcellulose), polyacrylamide, polyethylene oxide, polyethylene glycols, polypropylene glycols, polyvinyl alcohols, polyvinylpyrrolidones, starches and their modifications, polyacrylic acid and its homologs, polyacrylates, and copolymers and polymer blends of aforementioned polymers. Other such gelling agents are listed in Hand of Water-soluble Gums and Resins, eds. Crawford and Williams, (1980, McGraw-Hill, Inc.).

Other ingredients that may be added to the fungal growth medium include effective amounts of mold growth inhibitors (e.g., cycloheximide), antibacterial/antibiotics (e.g., penicillin, streptomycin, and choramphenical), boron, metals and metal containing compounds such as zinc, manganese, copper, iron, molybdenum, and magnesium oxide, P₂O₅, K₂O, those ingredients listed in Difco Manual - Dehydrated Culture Media and Reagents for Microbiology, (10^{th} Edition, 1984, Difco Laboratories, Detroit, MI).

Suitable fungal growth mediums are described in Difco Manual - Dehydrated Culture Media and Reagents for Microbiology, (10^{th} Edition, 1984, Difco Laboratories, Detroit, MI). Examples of such fungal growth mediums include Sabouraud's agar (glucose (2-4%), peptone (1%), agar (2%), and distilled water (q.s. 100%)) and Kimmig's agar (glucose (1%), peptone(0.5%), sodium chloride (0.5%), culture broth (1%), flake agar(3%), and distilled water (q.s. 100%)).

The fungal growth medium may be topically administered to the infection site as a solution, lotion, cream, shampoo, liposome formulation, spray, lacquer, or gel, or incorporated into a bandage to be affixed to the infection site as described below.

Examples of antifungal drugs include azoles (e.g., imidazoles) such as miconazole, econazole, ketoconazole, itraconazole, fluconazole, bifoconazole, terconazole, butaconazole, tioconazole, oxiconazole, sulconazole, saperconazole, clotrimazole, voriconazole, clotrimazole, and elubiol, allylamines such as terbinafine, morpholines such as amorolfine and naftifine, griseofulvin, undecylenic acid, haloprogin, butenafine, tolnaftate, nystatin, cyclohexamide, iodine, ciclopirox, tea tree oil, and pharmaceutically acceptable salts thereof.

The antifungal agent may comprise one or more pharmaceutically acceptable carriers as a pharmaceutical formulation. The carrier must be "acceptable" in the sense of being compatible with the antifungal and not deleterious to the subject to be treated. The antifungal formulation may be administered as an oral (e.g., a pill or capsule), parenteral (e.g., intravenous, intramuscular, or subcutaneous solution), or topical dosage form (e.g., topically administered to the infection site as a solution, lotion, cream, ointment, spray, lacquer, or gel, or incorporated into a bandage to be affixed to the infection site as described below). The antifungal agent may also be added to the topical fungal growth medium. Examples of topical dosage formulations (e.g., antifungal creams for skin and vaginal fungal infections, and shampoos to treat dandruff) are described below. U.S. Patent Nos. 4,775,678 and 5,514,698 describe antifungal cream compositions and manufacturing procedures. This invention can also be used to prepare antifungal diaper rash ointment comprising an antifungal agent (e.g., miconazole nitrate), fungal growth medium, fine zinc oxide particles, and petrolatum.

The dose of the antifungal agent for treating the fungal infections varies depending upon the type of fungal infection, the antifungal used, the manner of administration, and the age and body weight of the subject to be treated, and ultimately will be decided by the attending physician or veterinarian. Such amount of the antifungal is referred herein as a "therapeutically effective amount." In one embodiment, the antifungal formulation comprises between 0.01 and 10 percent (e.g., 0.5 to 5 percent), by weight, of the antifungal agent.

In one embodiment, the method further comprises the step of administering at least one keratin-softening agent capable of increasing penetration of the antifungal agent and/or fungal growth medium into fungus-infected keratinous tissues. The keratin-softening agent may be used to treat keratinous tissues (e.g., the nail) prior to or during application of the fungal growth medium and/or the antifungal agent. The keratin-softening agent may also be added to the fungal growth medium formulation and/or the antifungal agent formulation.

Examples of the keratinous tissues include nails, the stratum corneum of the skin, and the overgrowth of the skin's horny outer layer such as callus. Keratin-softening agents in the present invention include sulfur containing compounds, urea, salicylic acid, and a mixture thereof. According to the invention, the term "sulfur containing compounds" refers to (a) thio-compounds with one or more sulfhydryl functional groups capable of reacting with disulfide bonds of keratin, (b) thio-containing amino acids and their derivatives, and (c) certain sulfides. Examples of thio-compounds with one or more sulfhydryl functional groups capable of reacting with disulfide bonds of keratin thioglycolic acid and its salts (e.g., glycolates of calcium, sodium, strontium, potassium, ammonium, lithium, magnesium, and other metal salts), thioethylene glycol, thioglycerol, thioethanol, and thioactic acid, thiosalicylic acid, and their salts. Examples of thio-containing amino acids and their derivatives include the L- or D-isomers of cysteine, D-cysteine, N-acetyl-cysteine, homocysteine, cysteine methyl ester, cysteine ethyl ester, and N-carbamoyl cysteine, glutathion, and cysteamine. Examples of sulfides include calcium, sodium, potassium, lithium and strontium sulfides. U.S. Patent No. 5,696,164 describes the use of thio-containing amino acids and their derivatives to soften the nail for treatment of nail fungal infections.

When the sulfur containing compounds are thio-containing amino acids and their derivatives, the pH of the composition is preferably below pH 7 (e.g., below pH 5). Alternately, if the sulfur containing compounds are thio-compounds and sulfides, the pH of the composition is preferably above pH 6 (e.g., above pH 9). The concentration of a sulfur containing compound may range between 0.5 % to 20% (e.g., between 1% and 10%), by weight, of the administered composition. When the other keratin-softening agents urea and salicylic acid are used, the concentration of urea to be used ranges from 1% to 50% (e.g., from 5% to 40%, by weight, of the administered composition, and the concentration of salicylic acid to be used ranges from 0.1% to 40% (e.g., from 1 % to 20%), by weight, of the administered composition.

In one embodiment, the fungal growth medium, antifungal agent, and/or keratin-softening agent are incorporated into a bandage device to be affixed to the infection site (e.g., to provide desirable occlusion and protection from the accidental wiping-off of the agents). The bandage devices described in U.S. Patent Nos. 5,696,164, 5,181,914, and 5,814,031 may be used for this purpose, and is hereby incorporated by reference. Such devices are constructed in such a way that during the treatment, only the infection site is in direct contact with the agents. Thus, contact between the agents and the surrounding skin tissues is minimized in order to prevent skin irritation. In another embodiment, the agents of the present invention are incorporated in an adhesive layer, which is coated on a pliable polymeric backing sheet. This device resembles an adhesive tape, only with the treatment composition embedded in the adhesive layer.

In still another embodiment, the agents of the invention are incorporated in a hydrogel device where an aqueous solution or suspension of the agents are immobilized in a hydrogel layer, which is coated on a pliable polymeric backing sheet. During the application, the hydrogel layer is situated over the fungal infection site. The hydrogel device is secured to its position by the adhesion between the hydrogel layer and the infected tissue and/or surrounding skin tissue. Alternatively, the hydrogel device is secured by an adhesive layer coated on the polymeric backing sheet at the edges of the hydrogel device in a configuration commonly known in the art as an "island-type bandage". U.S. Patent Nos. 5,160,328, 5,181,914, 5,260,066 and 5,620,702 describe the basic composition and construction of such hydrogel devices that may be used according to the present invention.

In one embodiment, a bandage device with an absorbent pad (e.g., a gauze or non-woven pad) on an adhesive-coated backing layer, may also be used as a carrier device according to the present invention. See U.S. Patent Nos. 4,530,353, 4,549,653, 4,622,089, 5,633,070, and 5,814,031. The aforementioned hydrogel-bandages and absorbent-pad-bandages may be occlusive in order to retain the moisture and to facilitate permeation.

The topically infected tissues to be treated by the present invention may include the nail, skin, and mucosal membranes. Examples of fungal infections include those of the nails (e.g., onychomycosis), the skin (e.g., dermatomycosis), the mucosal membranes (e.g., fungal infections in vaginal and buccal membranes), the ear (e.g., the external auditory meatus- otomycosis), and the cornea (e.g., keratomycosis). Examples of skin fungal infections include teanea capitis, tinea nodosa, tinea axillaris, tinea barbae, tinea pedis, tinea versicolor, and dandruff which is associated with a yeast *pityrosporum ovale*.

The following is a description of a clinical application of the present invention and four compositions of the present invention. Other methods and compositions of the present invention can be conducted in an analogous manner by a person of ordinary skill in the art.

### Example 1:

A total of eight adult patients suffering from dermatophytic onychomycosis of the great toenail participated in the study. Their conditions had previously been unsuccessfully treated for months with fluconazole (Diflucan®, Pfizer, 100 mg/week), itraconazole (Sporanox®, Janssen, 400 mg/day, 1 wk./month), or terbinafine (Lamisil®, Novartis, 250 mg/day). In all cases, the disease recurred within six months after terminating the above oral treatment.

Each patient applied a 5% amorolfine nail lacquer (Loceryl®, Roche) once weekly. Every second day of the rest of the week, each patient applied a piece of Sabourand's agar (Mycoline bioMerieux, Marcy L'Etoile, France) to the affected nail. The agar was held in place for 24 hr with an adhesive (Hansamed®, Beiesdorf, Hamburg, Germany). The treatment was continued for one month, followed by a month or no treatment, and in some cases followed with another month of the same treatment.

Mycological cultures were performed 2, 4, and 6 months after treatment. Three of the patients (38%) were mycologically cured after the first month of treatment, four of the patients (50%) needed the second one-month treatment to be so cured, and only one patient (12%) failed to be so cured.

### Example 2: Miconazole Nitrate/Fungal Growth Medium Cream

A topical antifungal cream containing the antifungal miconazole nitrate and a fungal growth medium comprising glucose and peptone is manufactured by blending the following ingredients together:

| INGREDIENT | WEIGHT % |
|---|---|
| Miconazole Nitrate | 2.0 |
| Glucose | 2.0 |
| Peptone | 1.0 |
| Benzoic Acid | 0.2 |
| Butylated Hydroxyanisole | 0.1 |
| Mineral Oil (Heavy) | 3.0 |
| PEGLICOL-5-Oleate | 3.0 |
| PEGOXOL-7-Stearate | 20.0 |
| Purified Water | 68.7 |

### Example 3: Econazole Nitrate/Fungal Growth Medium Cream

A topical antifungal cream containing the antifungal econazole nitrate and a fungal growth medium comprising glucose and peptone is manufactured by blending the following ingredients together:

| INGREDIENT | WEIGHT % |
|---|---|
| Econazole Nitrate | 2.0 |
| Glucose | 2.0 |
| Peptone | 1.0 |
| Benzoic Acid | 0.2 |
| Butylated Hydroxyanisole | 0.1 |
| Mineral Oil | 3.0 |
| PEGLICOL-5-Oleate | 3.0 |
| PEGOXOL-7-Stearate | 20.0 |
| Purified Water | 69.7 |

### Example 4: Ketoconazole/Fungal Growth Medium Shampoo

An anti-dandruff shampoo containing the antifungal ketoconazole and a fungal growth medium comprising glucose and peptone is manufactured by blending the following ingredients together:

| INGREDIENT | WEIGHT % |
|---|---|
| Ketoconazole | 1.0 |
| Glucose | 2.0 |
| Peptone | 1.0 |
| Sodium Laureth Sulfate | 30.0 |
| Sodium Cocoyl Sarcosinate | 10.0 |
| Coconut Monoethanolamide | 4.0 |
| Ethylene Glycol Distearate | 1.25 |
| Acrylic Acid Copolymer (Carbomer 1342 from BF Goodrich) | 0.6 |
| Cosmetic Fragrance | 0.5 |
| Acrylatmide-DMDAAC Copolymer | 1.0 |
| Tetrasodium EDTA | 0.5 |
| 1-(3 Chloroallyl)-3,5,7-triaza-1-azoniaadamantane chloride (Quaternium-15) | 0.05 |
| Cosmetic colorant | 0.001 |
| Butylated Hydroxytoluene | 0.1 |
| Sodium Chloride | 0.25 |
| Purified Water | 47.5 |
| Sodium Hydroxide adjust to ≈pH 7 | 0.25 |

### Example 5: Antifungal Drug/Fungal Growth Medium Cream

A topical antifungal cream containing an antifungal agent and a fungal growth medium comprising glucose and peptone is manufactured by blending the following ingredients together:

| INGREDIENT | WEIGHT % |
|---|---|
| Antifungal Drug | 0.1-10.0 |
| Carbon Hydrate | 0.1-10.0 |
| Nitrogen-containing compound | 0.1-10.0 |
| Cetyl Alcohol | 1.0-7.0 |
| Stearyl Alcohol | 5.0-15.0 |
| Isopropyl Myristate | 1.0-5.0 |
| Propylene Glycol | 10.0-25.0 |
| Polysorbate 60 or 80 | 1.0-5.0 |
| Butylated Hydroxyanisole | 0.01-0.50 |
| Sodium or Potassium Hydroxide | Sufficient to adjust pH between 3-7 |
| Purified Water | q.s. 100 |

## Claims

1. The use of an antifungal agent for the manufacture of a medicament for the treatment of a topical fungus infection on a mammal, wherein an amount of a fungal growth medium effective to induce said fungus from the dormant state into the vegetative state is topically applied to said fungus-infacted tissue, and the fungal growth medium comprises a carbon hydrate.

2. The use of claim 1, wherein said fungal growth medium further comprises a nitrogen-containing compound.

3. The use of claims 1 or 2, wherein said carbon hydrate is selected from the group consisting of monosaccharides, disaccharides, polysaccharides, and starches, and said nitrogen-containing compound is selected from the group consisting of amino acids, peptides, polypeptides, proteins, and fragments thereof.

4. The use of claim 3, wherein said fungal growth medium comprises between 0.1 and 20 percent, by weight, of glucose, between 0.1 and 20 percent, by weight, of peptone, and water.

5. The use of claim 1, wherein said fungal growth medium further comprises a gelling agent selected from the group consisting of agar, gelatin, pectins, alginates, gums, hydrophilic cellulose polymers, polyacrylamide, polyethylene oxide, polyethylene glycols, polypropylene glycols, polyvinyl alcohols, polyvinylpyrrolidones, starches and its modifications, polyacrylic acid and its homologs, polyacrylates, and copolymers and polymer blends of aforementioned polymers.

6. The use of claim 1, wherein said antifungal is an azole, a morpholine, or an allylamine.

7. The use of claim 6, wherein said antifungal is selected from the group consisting of itraconazole, ketoconazole, miconazole, econazole, fluconazole, voriconazole, clotrimazole, amorolfine, terbinafine, naftifine, butenafine, ciclopirox, bifoconazole, terconazole, butaconazole, tioconazole, oxiconazole, sulconazole, saperconazole, elubiol, griseofulvin, undecylenic acid, haloprogin, tolnaftate, cyclohexamide, nystatin, iodine, tea tree oil and pharmaceutically acceptable salts thereof.

8. The use of claim 1, wherein said antifungal is administered during the administration of said fungal growth medium.

9. The use of claim 1, wherein said medicament is for administration in conjunction with with a keratin softening agent.

10. The use of claim 1, wherein said topical fungus infection is selected from the group consisting of onychomycosis, tinea capitis, tinea pedis, and tinea manuum.

11. A composition comprising a therapeutically effective amount of an antifungal agent and an amount of a fungal growth medium capable of inducing fungus from the dormant state into the vegetative state, wherein said fungal growth medium comprises a carbon hydrate.

12. A composition of claim 11, wherein said fungal growth medium further comprises a nitrogen-containing compound.

13. A composition of claims 11 or 12, wherein said carbon hydrate is selected from the group consisting of monosaccharides, disaccharides, polysaccharides, and starches, and said nitrogen-containing compound is selected from the group consisting of amino acids, peptides, polypeptides, proteins, and fragments thereof.

14. A composition of claim 13, wherein said fungal growth medium comprises between 0.1 and 20 percent, by weight, of glucose, between 0.1 and 20 percent, by weight, of peptone, and water.

15. A composition of claim 11, wherein said fungal growth medium further comprises a gelling agent selected from the group consisting of agar, gelatin, pectins, alginates, gums, hydrophilic cellulose polymers, polyacrylamide, polyethylene oxide, polyethylene glycols, polypropylene glycols, polyvinyl alcohols, polyvinylpyrrolidones, starches and its modifications, polyacrylic acid and its homologs, polyacrylates, and copolymers and polymer blends of aforementioned polymers.

16. A composition of claim 11, wherein said antifungal is an azole, a morpholine, or an allylamine.

17. A composition of claim 16, wherein said antifungal is selected from the group consisting of itraconazole, ketoconazole, miconazole, econazole, fluconazole, voriconazole, clotrimazole, amorolfine, terbinafine, naftifine, butenafine, ciclopirox, bifoconazole, terconazole, butaconazole, tioconazole, oxiconazole, sulconazole, saperconazole, elubiol, griseofulvin, undecylenic acid, haloprogin, tolnaftate, cyclohexamide, nystatin, iodine, tea tree oil and pharmaceutically acceptable salts thereof.

18. A composition of claim 11, wherein said composition further comprises a keratin-softening agent.

19. A bandage comprising an amount of a fungal growth medium capable of inducing fungus from the dormant state into the vegetative state, wherein said fungal growth medium comprises a carbon hydrate.

20. A bandage of claim 19, wherein said bandage further comprises a therapeutically effective amount of an antifungal agent.

## Patentansprüche

1. Verwendung eines Antipilzwirkstoffs zur Herstellung eines Medikaments zur Behandlung einer topischen Pilzinfektion bei einem Säugetier, wobei eine Menge eines Pilzwachstumsmediums, die wirksam ist, den Pilz zu veranlassen, vom Ruhezustand in den vegetativen Zustand überzutreten, topisch auf das mit dem Pilz infizierte Gewebe aufgetragen wird, und das Pilzwachstumsmedium einen Kohlenwasserstoff umfaßt.

2. Verwendung nach Anspruch 1, wobei das Pilzwachstumsmedium weiterhin eine Stickstoff-enthaltende Verbindung umfaßt.

3. Verwendung nach Anspruch 1 oder 2, wobei der Kohlenwasserstoff ausgewählt ist aus der Gruppe, bestehend aus Monosacchariden, Disacchariden, Polysacchariden und Stärken, und die Stickstoff-enthaltende Verbindung ausgewählt ist aus der Gruppe, bestehend aus Aminosäuren, Peptiden, Polypeptiden, Proteinen und Fragmenten davon.

4. Verwendung nach Anspruch 3, wobei das Pilzwachstumsmedium zwischen 0,1 und 20 Gewichtsprozent Glukose, zwischen 0,1 und 20 Gewichtsprozent Pepton und Wasser umfaßt.

5. Verwendung nach Anspruch 1, wobei das Pilzwachstumsmedium weiterhin ein Geliermittel umfaßt, das ausgewählt ist aus der Gruppe, bestehend aus Agar, Gelatine, Pektinen, Alginaten, Gummis, hydrophilen Zellulosepolymeren, Polyacrylamid, Polyethylenoxid, Polyethylenglykolen, Polypropylenglykolen, Polyvinylalkoholen, Polyvinylpyrrolidonen, Stärken und ihren Modifikationen, Polyacrylsäure und ihren Homologen, Polyacrylaten und Copolymeren und Polymermischungen der zuvor bezeichneten Polymeren.

6. Verwendung nach Anspruch 1, wobei der Antipilzwirkstoff ein Azol, ein Morpholin oder ein Allylamin ist.

7. Verwendung nach Anspruch 6, wobei der Antipilzwirkstoff ausgewählt ist aus der Gruppe, bestehend aus Itraconazol, Ketoconazol, Miconazol, Econazol, Fluconazol, Voriconazol, Clotrimazol, Amorolfin, Terbinafin, Naftifin, Butenafin, Ciclopirox, Bifoconazol, Terconazol, Butaconazol, Tioconazol, Oxiconazol, Sulconazol, Saperconazol, Elubiol, Griseofulvin, Undecensäure, Haloprogin, Tolnaftat, Cyclohexamid, Nystatin, Iod, Teebaumöl und pharmazeutisch zulässigen Salze davon.

8. Verwendung nach Anspruch 1, wobei der Antipilzwirkstoff während der Verabreichung des Pilzwachstumsmediums verabreicht wird.

9. Verwendung nach Anspruch 1, wobei das Medikament für eine Verabreichung in Verbindung mit einem Keratin-weichmachenden Wirkstoff vorgesehen ist.

10. Verwendung nach Anspruch 1, wobei die topische Pilzinfektion ausgewählt ist aus der Gruppe, bestehend aus Onychomycose, Tinea capitis, Tinea pedis und Tinea manuum.

11. Zusammensetzung, die eine therapeutisch wirksame Menge eines Antipilzwirkstoffs und eine Menge eines Pilzwachstumsmediums, die in der Lage ist, einen Pilz zu veranlassen, vom Ruhezustand in den vegetativen Zustand überzutreten, umfaßt, wobei das Pilzwachstumsmedium einen Kohlenwasserstoff umfaßt.

12. Zusammensetzung nach Anspruch 11, wobei das Pilzwachstumsmedium weiterhin eine Stickstoff-enthaltende Verbindung umfaßt.

13. Zusammensetzung nach Anspruch 11 oder 12, wobei der Kohlenwasserstoff ausgewählt ist aus Monosacchariden, Disacchariden, Polysacchariden und Stärken, und die Stickstoff-enthaltende Verbindung ausgewählt ist aus der Gruppe, bestehend aus Aminosäuren, Peptiden, Polypeptiden, Proteinen und Fragmenten davon.

14. Zusammensetzung nach Anspruch 13, wobei das Pilzwachstumsmedium zwischen 0,1 und 20 Gewichtsprozent Glukose, zwischen 0,1 und 20 Gewichtsprozent Pepton und Wasser umfaßt.

15. Zusammensetzung nach Anspruch 11, wobei das Pilzwachstumsmedium weiterhin ein Geliermittel umfaßt, das ausgewählt ist aus der Gruppe, bestehend aus Agar, Gelatine, Pektinen, Alginaten, Gummis, hydrophilen Zellulosepolymeren, Polyacrylamid, Polyethylenoxid, Polyethylenglykolen, Polypropylenglykolen, Polyvinylalkoholen, Polyvinylpyrrolidonen, Stärken und ihren Modifikationen, Polyacrylsäure und ihren Homologen, Polyacrylaten und Copolymeren und Polymermischungen der zuvor bezeichneten Polymeren.

16. Zusammensetzung nach Anspruch 11, wobei der Antipilzwirkstoff ein Azol, ein Morpholin oder ein Allylamin ist.

17. Zusammensetzung nach Anspruch 16, wobei der Antipilzwirkstoff ausgewählt ist aus der Gruppe, bestehend aus Itraconazol, Ketoconazol, Miconazol, Econazol, Fluconazol, Voriconazol, Clotrimazol, Amorolfin, Terbinafin, Naftifin, Butenafin, Ciclopirox, Bifoconazol, Terconazol, Butaconazol, Tioconazol, Oxiconazol, Sulconazol, Saperconazol, Elubiol, Griseofulvin, Undecensäure, Haloprogin, Tolnaftat, Cyclohexamid, Nystatin, Iod, Teebaumöl und pharmazeutisch zulässigen Salze davon.

18. Zusammensetzung nach Anspruch 11, wobei die Zusammensetzung weiterhin einen Keratin-weichmachenden Wirkstoff umfaßt.

19. Verband, der eine Menge eines Pilzwachstumsmediums umfaßt, die in der Lage ist, einen Pilz zu veranlassen, vom Ruhezustand in den vegetativen Zustand überzutreten, wobei das Pilzwachstumsmedium einen Kohlenwasserstoff umfaßt.

20. Verband nach Anspruch 19, wobei der Verband weiterhin eine therapeutisch wirksame Menge eines Antipilzwirkstoffs umfaßt.

## Revendications

1. Utilisation d'un agent antifongique pour la fabrication d'un médicament pour le traitement d'une infection fongique topique sur un mammifère, dans laquelle une quantité d'un milieu de croissance fongique efficace pour induire le passage du champignon de l'état dormant à l'état végétatif est appliqué par voie topique audit tissu infecté par un champignon, et le milieu de croissance fongique comprend un hydrate de carbone.

2. Utilisation de la revendication 1, dans laquelle ledit milieu de croissance fongique comprend en outre un composé contenant de l'azote.

3. Utilisation de la revendication 1 ou 2, dans lequel ledit hydrate de carbone est choisi dans le groupe consistant en monosaccharides, disaccharides, polysaccharides, et amidons, et ledit composé contenant de l'azote est choisi dans l'ensemble consistant en acides aminés, peptides, polypeptides, protéines, et leurs fragments.

4. Utilisation de la revendication 3, dans lequel ledit milieu de croissance fongique comprend entre 0,1 et 20 pour cent, en poids, de glucose, entre 0,1 et 20 pour cent, en poids, de peptone, et de l'eau.

5. Utilisation de la revendication 1, dans lequel le milieu de croissance fongique comprend en outre un agent gélifiant choisi dans l'ensemble consistant en agar, gélatine, pectines, alginates, gommes, polymères de cellulose hydrophiles, polyacrylamide, oxyde de polyéthylène, polyéthylèneglycols, polypropylèneglycols, alcools polyvinyliques, polyvinylpyrrolidones, amidons et leurs modifications, acide polyacrylique et ses homologues, polyacrylates et copolymères et mélanges de polymères des polymères mentionnés ci-dessus.

6. Utilisation de la revendication 1, dans laquelle ledit antifongique est un azole, une morpholine ou une allylamine.

7. Utilisation de la revendication 6, dans ledit antifongique est choisi dans l'ensemble consistant en itraconazole, kétoconazole, miconazole, éconazole, fluconazole, voriconazole, clotrimazole, amorolfine, terbinafine, naftifine, buténafine, ciclopirox, bifoconazole, terconazole, butaconazole, tioconazole, oxiconazole, sulconazole, saperconazole, elubiol, griséofulvine, acide undécylénique, haloprogine, tolnaftate, cyclohexamide, nystatine, iode, huile de Melaleuca à feuilles alternes et leurs sels pharmaceutiquement acceptables.

8. Utilisation de la revendication 1, dans laquelle ledit antifongique est administré pendant l'administration dudit milieu de croissance fongique.

9. Utilisation de la revendication 1, dans laquelle ledit médicament est destiné à l'administration en combinaison avec un agent ramollissant la kératine.

10. Utilisation de la revendication 1, dans laquelle ladite infection fongique topique est choisi dans l'ensemble consistant en onychomycose, teigne tondante microscopique, tinea pedis, tinea mannum.

11. Composition comprenant une quantité thérapeutiquement efficace d'un agent antifongique et une quantité d'un milieu de croissance fongique capable d'induire le passage du champignon de l'état dormant à l'état végétatif, ledit milieu de croissance fongique comprenant un hydrate de carbone.

12. Composition de la revendication 11, dans laquelle ledit milieu de croissance fongique comprend en outre un composé contenant de l'azote.

13. Composition de la revendication 11 ou 12, dans lequel ledit hydrate de carbone est choisi dans l'ensemble consistant en monosaccharides, disaccharides, polysaccharides, et amidons, et ledit composé contenant de l'azote est choisi dans le groupe consistant en acides aminés, peptides, polypeptides, protéines, et leurs fragments.

14. Composition de la revendication 13, dans laquelle ledit milieu de croissance fongique comprend entre 0,1 et 20 pour cent, en poids, de glucose, entre 0,1 et 20 pour cent, en poids, de peptone, et de l'eau.

15. Composition de la revendication 11, dans lequel le milieu de croissance fongique comprend en outre un agent gélifiant choisi dans l'ensemble consistant en agar, gélatine, pectines, alginates, gommes, polymères de cellulose hydrophiles, polyacrylamide, oxyde de polyéthylène, polyéthylèneglycols, polypropylèneglycols, alcools polyvinyliques, polyvinylpyrrolidones, amidons et leurs modifications, acide polyacrylique et ses homologues, polyacrylates et copolymères et mélanges de polymères des polymères mentionnés ci-dessus.

16. Composition de la revendication 11, dans laquelle ledit antifongique est un azole, une morpholine une allylamine.

17. Composition de la revendication 16, dans ledit antifongique est choisi dans l'ensemble consistant en itraconazole, kétoconazole, miconazole, éconazole, fluconazole, voriconazole, clotrimazole, amorolfine, terbinafine, naftifine, buténafine, ciclopirox, bifoconazole, terconazole, butaconazole, tioconazole, oxiconazole, sulconazole, saperconazole, elubiol, griséofulvine, acide undécylénique, haloprogine, tolnaftate, cyclohexamide, nystatine, iode, huile de Melaleuca à feuilles alternes et leurs sels pharmaceutiquement acceptables.

18. Composition de la revendication 11, ladite composition comprenant en outre un agent ramollissant la kératine.

19. Bandage comprenant une quantité d'un milieu de croissance fongique une quantité d'un milieu de croissance fongique capable d'induire le passage du champignon de l'état dormant à l'état végétatif, ledit milieu de croissance fongique contenant un hydrate de carbone.

20. Bandage de la revendication 19, ledit bandage comprenant en outre une quantité thérapeutiquement efficace d'un agent antifongique.
